# EUROPEAN PATENT APPLICATION

(11) **EP 3 273 245 A1**
(43) Date of publication of application: **24.01.2018**
(21) Application number: 16764985.4
(22) Date of filing: 15.03.2016
(51) Int. Cl.: G01N 33/68, G01N 33/48

(54) **EVALUATION METHOD, EVALUATION DEVICE, EVALUATION PROGRAM, EVALUATION SYSTEM, AND TERMINAL DEVICE**

(30) Priority: 16.03.2015 JP 2015052432
(71) Applicant: Ajinomoto Co., Inc., Tokyo 104-8315 (JP)
(72) Inventor: HISAMATSU, Tadakazu, Tokyo 160-8582 (JP); KANAI, Takanori, Tokyo 160-8582 (JP); HIBI, Toshifumi, Tokyo 160-8582 (JP); ONO, Nobukazu, Kawasaki-shi Kanagawa 210-8681 (JP); IMAIZUMI, Akira, Kawasaki-shi Kanagawa 210-8681 (JP); HASHIMOTO, Masaki, Kawasaki-shi Kanagawa 210-8681 (JP); MORI, Maiko, Kawasaki-shi Kanagawa 210-8681 (JP); UO, Michihide, Kawasaki-shi Kanagawa 210-8681 (JP)
(74) Representative: Dunne, Paul David
(86) International application number: PCT/JP2016/058210
(87) International publication number: WO 2016/148155

(57) **Abstract**

Provided is the evaluating method and the like, which can provide reliable information that may be helpful in knowing the future risk of relapse of ulcerative colitis. In the present embodiment, (i) the concentration data on the concentration value of histidine, albumin, hemoglobin, or glutamic acid in blood of the subject with ulcerative colitis in a remission phase is obtained and (ii) the future risk of relapse of ulcerative colitis for the subject is evaluated using the concentration value of histidine, albumin, hemoglobin, or glutamic acid included in the obtained concentration data of the subject.

## Description

### TECHNICAL FIELD

The present invention relates to an evaluating method, an evaluating apparatus, an evaluating program product, an evaluating system, and a terminal apparatus.

### BACKGROUND ART

Inflammatory bowel disease (IBD) is a collective term of bowel disease with inflammation. Examples of major disease of IBD include ulcerative colitis (UC) and Crohn's disease (CD).

UC is a chronic colitis of unknown origin causing ulceration or erosion in the large bowel, and shows symptoms, such as bloody stool or diarrhea, frequently throughout the day. Those symptoms repeat an active phase worsening inflammation and a remission phase healing inflammation. In an active phase, a patient may need hospital treatment. Furthermore, radical treatment for UC is not found. In addition, a patient in a remission phase always has a risk of relapse.

As an index for measuring UC activity, pathological score, such as a clinical activity index (CAI) has been used (for example, Lichtiger CAI, see Non-patent Literature 1). This pathological score is mainly used for distinguishing an active phase and a remission phase and determining severity. Note that, CAI is an index based on the summation (aggregation) of scored conditions, such as abdominal symptom including abdominal pain or other signs, the number of diarrheas and bloody stools, and degrees of general symptoms.

The degrees of abdominal pain or the points of general symptoms, however, depend on subjective decision. In addition, correlation among the points of corresponding items is not clear. Thus, in a remission phase, it is difficult to accurately compare the states of disease of a patient. Furthermore, depending on the points of current pathological score alone, it is difficult to determine whether the disease will progress from a remission phase to an active phase, that is, whether the disease is prone to relapse. For this reason, predicting the degrees of relapse in a remission phase of UC is considered to be valuable to a treatment for preventing UC from relapsing, and an objective marker index is desired as a predictive index of relapse in a remission phase.

Some predictive indexes of relapse have been studied up to now. One of the indexes is based on observation of intestinal mucosa using a colonoscopy, and it has been reported that microdefect in mucosa relates to relapse (see Non-Patent Literature 2). In addition, the measurement of calprotectin in stools has been approved in diagnoses and monitoring of IBD (see Non-Patent Literature 3). Furthermore, it has been suggested that the measurement of calprotectin in stools has potential to predict relapse. Calprotectin is a protein derived from neutrophils, and it has been reported that calprotectin concentration in stool reflects the degrees of inflammation.

Incidentally, an amino acid concentration in plasma is regulated by homeostasis, and a healthy person has a certain amino acid concentration profile. In contrast, it has been known that an amino acid concentration profile in plasma of a patient having an organ disease in the liver, a kidney or other organ, suffering from cancer, or being in a state of metabolic syndrome represented by impaired glucose tolerance is changed from a normal state. In particular, Patent Document 1 relating to evaluation of IBD has been published. Furthermore, Patent Documents 2 to 4 relating to a method of associating an amino acid concentration with a biological state have been published.

### PRIOR ART REFERENCES

### PATENT DOCUMENT

Patent Document 1: WO 2008/090941
Patent Document 2: WO 2004/052191
Patent Document 3: WO 2006/098192
Patent Document 4: WO 2009/054351

### NON-PATENT LITERATURE

Non-Patent Literature 1: N Engl J Med. 1994, Jun 30;330(26):1841-5.
Non-Patent Literature 2: Gastrointest Endosc. 2002 Oct;56(4):535-42.
Non-Patent Literature 3: Am J Gastroenterol. 2007 Apr;102(4):803-13. Epub 2007 Feb 23.

### DISCLOSURE OF INVENTION

### PROBLEM TO BE SOLVED BY THE INVENTION

However, an examination using a colonoscopy spends a time for pretreatment, for example, an intestinal lavage. Furthermore, usually, acceptability to insertion of a colonoscopy is not high, and thus more convenient examining method has been desired. With respect to the measurement of calprotectin, a patient is required to handle stools, and therefore feels unpleasant. Furthermore, average sampling of stools and solubilization of the sample are needed in examination, whereby the test values are not necessarily correlated with the states of disease and possibility of relapse accurately.

In addition, it is difficult to predict the relapse of UC with the techniques disclosed in Patent Documents 1 to 4.

That is, there is no index for predicting relapse of UC using blood obtained by sampling that is easy and less-invasive. Note that, capability of predicting relapse of UC of CRP (C-reactive protein) used as an inflammation index for various diseases has not been reported.

The present invention has been made in view of the problems described above, and an object of the present invention is to provide an evaluating method, an evaluating apparatus, an evaluating program product, an evaluating system, and a terminal apparatus, which can provide reliable information that may be helpful in knowing a future risk of relapse of UC.

### MEANS FOR SOLVING PROBLEMS

To solve the problem and achieve the object described above, an evaluating method according to one aspect of the present invention includes an evaluating step of evaluating a future risk of relapse of ulcerative colitis (hereinafter may be simply referred to as risk of relapse) for a subject to be evaluated having ulcerative colitis in a remission phase using a concentration value of histidine (hereinafter may be referred to as His), albumin (hereinafter may be referred to as Alb), hemoglobin (hereinafter may be referred to as Hb), or glutamic acid (hereinafter may be referred to as Glu) in blood of the subject.

The evaluating method according to another aspect of the present invention is the evaluating method, wherein the evaluating step uses the concentration values of histidine and albumin, histidine and hemoglobin, or albumin and hemoglobin.

The evaluating method according to still another aspect of the present invention is the evaluating method, wherein the evaluating step uses the concentration values of histidine, albumin, and hemoglobin.

The evaluating method according to still another aspect of the present invention is the evaluating method, wherein the evaluating step evaluates the future risk of relapse of ulcerative colitis for the subject by calculating a value of a formula (hereinafter may be referred to as value of evaluation formula, or referred to as evaluation value) further using the formula (hereinafter may be referred to as evaluation formula) including an explanatory variable to be substituted with the concentration value of histidine, albumin, or hemoglobin.

The evaluating method according to still another aspect of the present invention is the evaluating method, wherein the formula includes the explanatory variables to be substituted with the concentration values of histidine and albumin, histidine and hemoglobin, or albumin and hemoglobin.

The evaluating method according to still another aspect of the present invention is the evaluating method, wherein the formula includes the explanatory variables to be substituted with the concentration values of histidine, albumin, and hemoglobin.

An evaluating apparatus according to one aspect of the present invention is an evaluating apparatus including a control unit. The control unit includes an evaluating unit that evaluates a future risk of relapse of ulcerative colitis for a subject to be evaluated having ulcerative colitis in a remission phase using a concentration value of histidine, albumin, hemoglobin, or glutamic acid in blood of the subject.

An evaluating method according to one aspect of the present invention is an evaluating method executed by an information processing apparatus including a control unit. The evaluating method includes an evaluating step of evaluating a future risk of relapse of ulcerative colitis for a subject to be evaluated having ulcerative colitis in a remission phase using a concentration value of histidine, albumin, hemoglobin, or glutamic acid in blood of the subject. The evaluating step is executed by the control unit.

An evaluating program product according to one aspect of the present invention is an evaluating program product having a non-transitory tangible computer readable medium including programmed instructions for making an information processing apparatus including a control unit execute an evaluating method. The evaluating method includes an evaluating step of evaluating a future risk of relapse of ulcerative colitis for a subject to be evaluated having ulcerative colitis in a remission phase using a concentration value of histidine, albumin, hemoglobin, or glutamic acid in blood of the subject. The evaluating step is executed by the control unit.

A recording medium according to one aspect of the present invention is a non-transitory tangible computer-readable recording medium including the programmed instructions for making an information processing apparatus execute the evaluating method.

[**0024**] An evaluating system according to one aspect of the present invention is an evaluating system including an evaluating apparatus including a control unit and a terminal apparatus including a control unit to provide concentration data on a concentration value of histidine, albumin, hemoglobin, or glutamic acid in blood of a subject to be evaluated having ulcerative colitis in a remission phase that are connected to each other communicatively via a network. The control unit of the terminal apparatus includes a concentration data-sending unit that transmits the concentration data of the subject to the evaluating apparatus and a result-receiving unit that receives an evaluation result on a future risk of relapse of ulcerative colitis for the subject, transmitted from the evaluating apparatus. The control unit of the evaluating apparatus includes a concentration data-receiving unit that receives the concentration data of the subject transmitted from the terminal apparatus, an evaluating unit that evaluates the future risk of relapse of ulcerative colitis for the subject using the concentration value of histidine, albumin, hemoglobin, or glutamic acid included in the concentration data of the subject received by the concentration data-receiving unit, and a result-sending unit that transmits the evaluation result obtained by the evaluating unit to the terminal apparatus.

A terminal apparatus according to one aspect of the present invention is a terminal apparatus including a control unit. The control unit includes a result-obtaining unit that obtains an evaluation result on a future risk of relapse of ulcerative colitis for a subject to be evaluated having ulcerative colitis in a remission phase. The evaluation result is the result of evaluating the future risk of relapse of ulcerative colitis for the subject using a concentration value of histidine, albumin, hemoglobin, or glutamic acid in blood of the subject.

The terminal apparatus according to another aspect of the present invention is the terminal apparatus, wherein the apparatus is communicatively connected via a network to an evaluating apparatus that evaluates the future risk of relapse of ulcerative colitis for the subject. The control unit further includes a concentration data-sending unit that transmits concentration data on the concentration value of histidine, albumin, hemoglobin, or glutamic acid of the subject to the evaluating apparatus. The result-obtaining unit receives the evaluation result transmitted from the evaluating apparatus.

An evaluating apparatus according to one aspect of the present invention is an evaluating apparatus including a control unit, being connected communicatively via a network to a terminal apparatus that provides concentration data on a concentration value of histidine, albumin, hemoglobin, or glutamic acid in blood of a subject to be evaluated having ulcerative colitis in a remission phase. The control unit includes a concentration data-receiving unit that receives the concentration data of the subject transmitted from the terminal apparatus, an evaluating unit that evaluates a future risk of relapse of ulcerative colitis for the subject using the concentration value of histidine, albumin, hemoglobin, or glutamic acid included in the concentration data of the subject received by the concentration data-receiving unit, and a result-sending unit that transmits an evaluation result obtained by the evaluating unit to the terminal apparatus.

### EFFECTS OF THE INVENTION

According to the present invention, the future risk of relapse of ulcerative colitis for the subject having ulcerative colitis in a remission phase is evaluated using the concentration value of His, Alb, Hb, or Glu in blood of the subject. Thus, the present invention achieves the effect of being able to provide reliable information that may be helpful in knowing the future risk of relapse of ulcerative colitis. Furthermore, the present invention achieves the effect of being able to start active prevention of relapse with medicines at an appropriate timing.

### BRIEF DESCRIPTION OF THE DRAWINGS

FIG. 1 is a principle configurational diagram showing a basic principle of a first embodiment;
FIG. 2 is a principle configurational diagram showing a basic principle of a second embodiment;
FIG. 3 is a diagram showing an example of an entire configuration of a present system;
FIG. 4 is a diagram showing another example of an entire configuration of the present system;
FIG. 5 is a block diagram showing an example of a configuration of an evaluating apparatus 100 in the present system;
FIG. 6 is a chart showing an example of information stored in a user information file 106a;
FIG. 7 is a chart showing an example of information stored in a concentration data file 106b;
FIG. 8 is a chart showing an example of information stored in an index state information file 106c;
FIG. 9 is a chart showing an example of information stored in a designated index state information file 106d;
FIG. 10 is a chart showing an example of information stored in an evaluation formula file 206e1;
FIG. 11 is a chart showing an example of information stored in an evaluation result file 106f;
FIG. 12 is a block diagram showing a configuration of an evaluating part 102i;
FIG. 13 is a block diagram showing an example of a configuration of a client apparatus 200 in the present system;
FIG. 14 is a block diagram showing an example of a configuration of a database apparatus 400 in the present system;
FIG. 15 is a table of results of Cox regression p values and standardized regression coefficients of amino acids and other substances; and
FIG. 16 is a table of results of comparing the capabilities of predicting relapse for the indexes and the combinations.

### BEST MODE(S) FOR CARRYING OUT THE INVENTION

Hereinafter, an embodiment (first embodiment) of the evaluating method according to the present invention and an embodiment (second embodiment) of the evaluating apparatus, the evaluating method, the evaluating program product, the evaluating system, and the terminal apparatus according to the present invention are described in detail with reference to the drawings. The present invention is not limited to these embodiments. For example, the present embodiments may be applied to an evaluation of a future risk of relapse of inflammatory bowel disease or Crohn's disease.

### First Embodiment

### 1-1. Outline of first embodiment

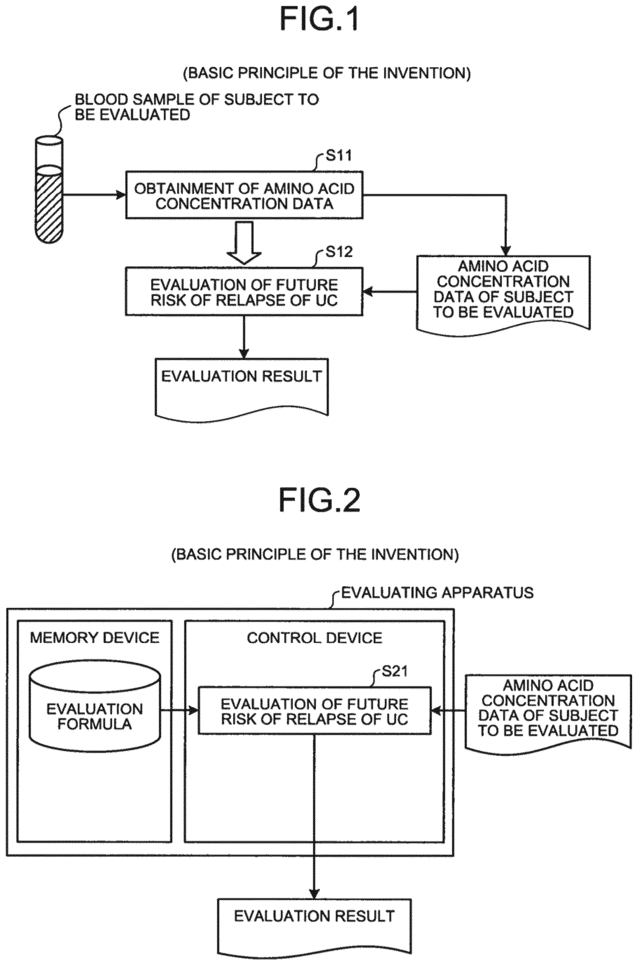

Here, an outline of the first embodiment will be described with reference to FIG. 1. FIG. 1 is a principle configurational diagram showing a basic principle of the first embodiment.

First, concentration data on a concentration value of a substance (a substance in blood including at least one of His, Alb, Hb, and Glu) contained in the blood (including, for example, plasma or serum) extracted from a subject to be evaluated in a remission phase of UC (for example, an individual animal or human in a remission phase of UC) is obtained (Step S11).

At step S11, for example, the concentration data on the substance in blood measured by a company or other organizations that measures concentration values may be obtained. In addition, for example, the following measuring method of (A), (B), or (C) may be used to measure the concentration values of the amino acids such as His and Glu from the blood sampled from the subject to obtain the concentration data on the concentration values of the amino acids such as His and Glu. For example, the measuring method of (C) may be used to measure the concentration values of Alb and Hb from the blood sampled from the subject to obtain the concentration data on the concentration values of Alb and Hb. Here, the unit of the concentration values of the substances in blood may be molar concentration, weight concentration, enzyme activity, or one obtained by addition, subtraction, multiplication, and division of any constant with these concentrations.
(A) Plasma is separated from blood by centrifuging the collected blood sample. All plasma samples are frozen and stored at -80°C until the amino acid concentration value is measured. At the time of measuring the amino acid concentration value, acetonitrile is added to perform a protein removal treatment, pre-column derivatization is then performed using a labeled reagent (3-aminopyridyl-N-hydroxysuccinimidyl carbamate), and the amino acid concentration value is analyzed by liquid chromatograph mass spectrometer (LC/MS) (see International Publication WO 2003/069328 and International Publication WO 2005/116629).
(B) Plasma is separated from blood by centrifuging the collected blood sample. All plasma samples are frozen and stored at -80°C until the amino acid concentration value is measured. At the time of measuring the amino acid concentration value, sulfosalicylic acid is added to perform a protein removal treatment, and the amino acid concentration value is analyzed by an amino acid analyzer based on post-column derivatization using a ninhydrin reagent.
(C) Blood cell separation is performed on the collected blood sample by using a membrane, MEMS (Micro Electro Mechanical Systems) technology, or the principle of centrifugation, whereby plasma or serum is separated from the blood. A plasma or serum sample the concentration value of which is not measured immediately after obtaining the plasma or the serum is frozen and stored at -80°C until the concentration value is measured. At the time of measuring the concentration value, a molecule that reacts with or binds to a target substance in blood, such as an enzyme or an aptamer, and the like are used to perform quantitative analysis and the like on an increasing or decreasing substance or a spectroscopic value by substrate recognition, whereby the concentration value is analyzed.

The risk of relapse for the subject is evaluated using, as the evaluation value for evaluating the risk of relapse, the concentration value of at least one of His, Alb, Hb, and Glu (any one or more of His, Alb, Hb, and Glu) included in the concentration data obtained at step S11 (step S12). Before step S12 is executed, data such as defective and outliers may be removed from the concentration data obtained at step S11.

According to the first embodiment described above, the concentration data of the subject is obtained at step S11, and at step S12, the risk of relapse for the subject is evaluated using, as the evaluation value, the concentration value of at least one of His, Alb, Hb, and Glu included in the concentration data of the subject obtained at step S11. Hence, reliable information that may be helpful in knowing the risk of relapse can be provided.

It may be decided that the concentration value of at least one of His, Alb, Hb, and Glu reflects the risk of relapse for the subject. The concentration value may be converted, for example, by the methods listed below, and it may be decided that the converted value reflects the risk of relapse for the subject. In other words, the concentration value or the converted value may be treated per se as an evaluation result on the risk of relapse for the subject.

The concentration value may be converted such that a possible range of the concentration value falls within a predetermined range (for example, the range from 0.0 to 1.0, the range from 0.0 to 10.0, the range from 0.0 to 100.0, or the range from -10.0 to 10.0), for example, by addition, subtraction, multiplication, and division of any given value with the concentration value, by conversion of the concentration value by a predetermined conversion method (for example, index transformation, logarithm transformation, angular transformation, square root transformation, probit transformation, reciprocal transformation, Box-Cox transformation, or power transformation), or by performing a combination of these computations on the concentration value. For example, a value of an exponential function with the concentration value as an exponent and Napier constant as the base may be further calculated (specifically, a value of p/(1-p) where a natural logarithm ln(p/(1-p)) is equal to the concentration value when the probability p that the risk of relapse has a predetermined state (for example, a state of exceeding a criterion value) is defined), and a value (specifically, a value of the probability p) may be further calculated by dividing the calculated value of the exponential function by the sum of 1 and the value of the exponential function.

The concentration value may be converted such that the converted value is a particular value when a particular condition is met. For example, the concentration value may be converted such that the converted value is 5.0 when the sensitivity is 95% and the converted value is 8.0 when the sensitivity is 80%.

For each of His, Alb, Hb, and Glu, after normally distributing the concentration distribution, the concentration value may be standardized with a mean of 50 and a standard deviation of 10.

These conversions may be performed by gender or age.

Positional information about a position of a predetermined mark on a predetermined scale visually presented on a display device such as a monitor or a physical medium such as paper may be generated using the concentration value of at least one of His, Alb, Hb, and Glu or, if the concentration value is converted, the converted value, and it may be decided that the generated positional information reflects the risk of relapse for the subject. The predetermined scale is for evaluating the risk of relapse and is, for example, a graduated scale at least marked with graduations corresponding to the upper limit value and the lower limit value in a possible range of the concentration value or the converted value, or part of the range. The predetermined mark corresponds to the concentration value or the converted value and is, for example, a circle sign or a star sign.

If the concentration value of at least one of His, Alb, Hb, and Glu is lower than a predetermined value (e.g., mean ±1SD, 2SD, 3SD, N quantile, N percentile, or a cutoff value the clinical significance of which is recognized) or is equal to or lower than the predetermined value, or the concentration is equal to or higher than the predetermined value or is higher than the predetermined value, the risk of relapse for the subject may be evaluated. In this case, instead of the concentration itself, a concentration standard score (a value obtained by normally distributing the concentration distribution by gender and then standardizing the concentration value with a mean of 50 and a standard deviation of 10 for each of His, Alb, Hb, and Glu) may be used. For example, if the concentration standard score is lower than the mean -2SD (when the concentration standard score<30) or if the concentration standard score is higher than the mean +2SD (when the concentration standard score>70), the risk of relapse for the subject may be evaluated.

The risk of relapse for the subject may be evaluated by calculating a value of a formula using the concentration value of at least one of His, Alb, and Hb and the formula including an explanatory variable to be substituted with the concentration value of at least one of His, Alb, and Hb.

It may be decided that the calculated value of the formula reflects the risk of relapse for the subject. The value of the formula may be converted, for example, by the methods listed below, and it may be decided that the converted value reflects the risk of relapse for the subject. In other words, the value of the formula or the converted value may be treated per se as the evaluation result on the risk of relapse for the subject.

The value of the evaluation formula may be converted such that a possible range of the value of the evaluation formula falls within the predetermined range (for example, the range from 0.0 to 1.0, the range from 0.0 to 10.0, the range from 0.0 to 100.0, or the range from -10.0 to 10.0), for example, by addition, subtraction, multiplication, and division of any given value with the value of the evaluation formula, by conversion of the value of the evaluation formula by the predetermined conversion method (for example, the index transformation, the logarithm transformation, the angular transformation, the square root transformation, the probit transformation, the reciprocal transformation, the Box-Cox transformation, or the power transformation), or by performing a combination of these computations on the value of the evaluation formula. For example, a value of an exponential function with the value of the evaluation formula as an exponent and Napier constant as the base may be further calculated (specifically, a value of p/(1-p) where a natural logarithm ln(p/(1-p)) is equal to the value of the evaluation formula when the probability p that the risk of relapse has the predetermined state (for example, the state of exceeding the criterion value) is defined), and the value (specifically, the value of the probability p) may be further calculated by dividing the calculated value of the exponential function by the sum of 1 and the value of the exponential function.

The value of the evaluation formula may be converted such that the converted value is a particular value when a particular condition is met. For example, the value of the evaluation formula may be converted such that the converted value is 5.0 when the sensitivity is 95% and the converted value is 8.0 when the sensitivity is 80%.

The value of the evaluation formula may be standardized with a mean of 50 and a standard deviation of 10.

These conversions may be performed by gender or age.

The evaluation value in the present description may be the value of the evaluation formula per se or may be the the converted value of the value of the evaluation formula.

The positional information about the position of the predetermined mark on the predetermined scale visually presented on the display device such as the monitor or the physical medium such as the paper may be generated using the value of the formula or, if the value of the formula is converted, the converted value, and it may be decided that the generated positional information reflects the risk of relapse for the subject. The predetermined scale is for evaluating the risk of relapse and is, for example, the graduated scale at least marked with the graduations corresponding to the upper limit value and the lower limit value in a possible range of the value of the formula or the converted value, or part of the range. The predetermined mark corresponds to the value of the formula or the converted value and is, for example, the circle sign or the star sign.

The degree of the risk of relapse for the subject may be qualitatively evaluated. Specifically, the subject may be classified into any one of a plurality of categories defined at least considering the degree of the risk of relapse, using "the concentration value of at least one of His, Alb, Hb, and Glu and one or more preset thresholds" or "the concentration value of at least one of His, Alb, and Hb, the formula including the explanatory variable to be substituted with the concentration value of at least one of His, Alb, and Hb, and one or more preset thresholds". The categories may include a category to which a subject whose risk of relapse (the degree of the possibility of having relapse of UC in the future) is high belongs, a category to which a subject whose risk of relapse is low belongs, and a category to which a subject whose risk of relapse is intermediate belongs. The categories may include the category to which the subject whose risk of relapse is high belongs and the category to which the subject whose risk of relapse is low belongs. The concentration value or the value of the formula may be converted by the predetermined method, and the subject may be classified into any one of the categories using the converted value.

The form of the expression is not specifically designated, however, for example, may be any one of the following expressions: (1) a linear model such as a multiple regression equation, a linear discriminant, a principal component analysis, and a canonical discriminant analysis that are based on the least-squares method; (2) a generalized linear model such as a logistic regression and a Cox regression that are based on a maximum likelihood method; (3) a generalized linear mixed model considering random effects due to individual differences, facility differences, and other factors in addition to the generalized linear model; (4) an expression generated by a cluster analysis, such as the K-means method and a hierarchical cluster analysis; (5) an expression generated on the basis of the Bayesian statistics such as the Markov chain Monte Carlo (MCMC), the Bayesian network, and the hierarchical Bayesian method; (6) an expression generated by a class classification such as a support vector machine and a decision tree; (7) an expression generated by a method such as a fractional expression that does not belong to the above-cited categories; and (8) an expression represented as, for example, the summation of expressions of different forms.

The formula employed as the evaluation formula may be prepared by a method described in WO 2004/052191 that is an international application filed by the present applicant or by a method described in WO 2006/098192 that is an international application filed by the present applicant. Any formulae obtained by these methods can be preferably used in the evaluation of the risk of relapse, regardless of the unit of the concentration value of the substance in blood including at least one of His, Alb, Hb, and Glu in the concentration data as input data.

In the multiple regression equation, the multiple logistic regression equation, and the canonical discriminant function, a coefficient and a constant term are added to each explanatory variable, and the coefficient and the constant term may be preferably real numbers, more preferably values in the range of 99% confidence interval for the coefficient and the constant term obtained from data for the various kinds of classifications described above, more preferably values in the range of 95% confidence interval for the coefficient and the constant term obtained from data for the various kinds of classifications described above. The value of each coefficient and the confidence interval thereof may be those multiplied by a real number, and the value of the constant term and the confidence interval thereof may be those having an arbitrary actual constant added or subtracted or those multiplied or divided by an arbitrary actual constant. When an expression such as the logistic regression, the linear discriminant, and the multiple regression equation is used as the evaluation formula, a linear transformation of the expression (addition of a constant and multiplication by a constant) and a monotonic increasing (decreasing) transformation (for example, a logit transformation) of the expression do not alter evaluation performance and thus evaluation performance after transformation is equivalent to that before transformation. Therefore, the expression includes an expression that is subjected to the linear transformation and the monotonic increasing (decreasing) transformation.

In the fractional expression, the numerator of the fractional expression is expressed by the sum of the explanatory variables A, B, C etc. and the denominator of the fractional expression is expressed by the sum of the explanatory variables a, b, c etc. The fractional expression also includes the sum of the fractional expressions α, β, γ etc. (for example, α+β) having such constitution. The fractional expression also includes divided fractional expressions. The explanatory variables used in the numerator or denominator may have suitable coefficients respectively. The explanatory variables used in the numerator or denominator may appear repeatedly. Each fractional expression may have a suitable coefficient. A value of a coefficient for each explanatory variable and a value for a constant term may be any real numbers. In a fractional expression and the one in which explanatory variables in the numerator and explanatory variables in the denominator in the fractional expression are switched with each other, the positive and negative signs are generally reversed in correlation with objective explanatory variables, but because their correlation is maintained, the evaluation performance can be assumed to be equivalent. The fractional expression therefore also includes the one in which explanatory variables in the numerator and explanatory variables in the denominator in the fractional expression are switched with each other.

When the furure risk of relapse of UC is evaluated, a value related to other biological information (for example, values listed below) may further be used in addition to the concentration values of the amino acids including His and Glu, Alb, and Hb. The formulae adopted as the evaluation formula may additionally include one or more explanatory variables to be substituted with the value related to other biological information (for example, values listed below) in addition to the explanatory variables to be substituted with the concentration values of the amino acids including His and Glu, Alb, and Hb.
1. Concentration values of metabolites in blood other than amino acids (e.g., amino acid metabolites, carbohydrates, and lipids), proteins, peptides, minerals, hormones, and the like.
2. Blood test values such as total protein, triglyceride (neutral fat), HbAlc, glycoalbumin, insulin resistance index, total cholesterol, LDL cholesterol, HDL cholesterol, amylase, total bilirubin, creatinine, estimated glomerular filtration rate (eGFR), uric acid, GOT (AST), GPT (ALT), GGTP (γ-GTP), glucose (glucose level), CRP (C-reactive protein), erythrocyte, hematocrit, MCV, MCH, MCHC, leucocyte, and the number of thrombocytes.
3. Values obtained from image information such as ultrasonic echo, X ray, CT (Computer Tomography), MRI (Magnetic Resonance Imaging), and endoscope image.
4. Values of biological indices such as age, height, weight, BMI, abdominal girth, systolic blood pressure, diastolic blood pressure, gender, smoking information, dietary information, drinking information, exercise information, stress information, sleeping information, family medical history information, and disease history information (for example, diabetes).

### Second Embodiment

### 2-1. Outline of the second embodiment

Here, outlines of the second embodiment will be described in detail with reference to FIG. 2. FIG. 2 is a principle configurational diagram showing a basic principle of the second embodiment. In the description of the present second embodiment, description duplicating that of the first embodiment is sometimes omitted. In particular, herein, when the risk of relapse is evaluated, a case of using the value of the evaluation formula or the converted value thereof is described as one example. However, for example, the concentration value of at least one of His, Alb, Hb, and Glu or the converted value thereof (for example, the concentration standard score) may be used.

A control device evaluates the risk of relapse for the subject by calculating the value of the formula using (i) the concentration value of at least one of His, Alb, and Hb included in the previously obtained concentration data of the subject in a remission phase of UC (for example, an individual animal or human in a remission phase of UC) on the concentration value of the substance in blood including at least one of His, Alb, and Hb in blood and (ii) the formula previously stored in a memory device including the explanatory variable to be substituted with the concentration value of at least one of His, Alb, and Hb (step S21).

According to the second embodiment described above, at step S21, the risk of relapse for the subject is evaluated by calculating the value of the evaluation formula using (i) the concentration value of at least one of His, Alb, and Hb included in the concentration data of the subject and (ii) the formula stored in the memory device as the evaluation formula, including the explanatory variable to be substituted with the concentration value of at least one of His, Alb, and Hb. Hence, reliable information that may be helpful in knowing the risk of relapse can be provided.

Here, the summary of the evaluation formula-preparing processing (steps 1 to 4) is described in detail. The processing described below is merely one example, and the method of preparing the evaluation formula is not limited thereto.

First, the control device prepares a candidate formula (e.g., y=a₁x₁+a₂x₂+...+aₙxₙ, y: index data, x₁: concentration data, aᵢ: constant, i = 1,2, ..., n) that is a candidate for the evaluation formula, based on a predetermined formula-preparing method from index state information previously stored in the memory device containing the concentration data and index data on a state of a predictive index of relapse of UC (step 1). Data containing defective and outliers may be removed in advance from the index state information.

In step 1, a plurality of the candidate formulae may be prepared from the index state information by using a plurality of the different formula-preparing methods (including those for multivariate analysis such as the principal component analysis, the discriminant analysis, the support vector machine, the multiple regression analysis, the Cox regression analysis, the logistic regression analysis, the K-means method, the cluster analysis, and the decision tree). Specifically, a plurality of groups of the candidate formulae may be prepared simultaneously and concurrently by using a plurality of different algorithms with the index state information which is multivariate data composed of the concentration data and the index data obtained by analyzing blood obtained from a large number of groups of patients in a remmition phase of UC and groups having the predictive index of relapse of UC of being a predetermined state (for example, a state of exceeding a criterion value). For example, the two different candidate formulae may be formed by performing the discriminant analysis and the logistic regression analysis simultaneously with the different algorithms. Alternatively, the candidate formula may be formed by converting the index state information with the candidate formula prepared by performing the principal component analysis and then performing the discriminant analysis of the converted index state information. In this way, it is possible to finally prepare the most suitable evaluation formula.

The candidate formula prepared by the principal component analysis is a linear expression including each explanatory variable maximizing the variance of all concentration data. The candidate formula prepared by the discriminant analysis is a high-powered expression (including exponential and logarithmic expressions) including each explanatory variable minimizing the ratio of the sum of the variances in respective groups to the variance of all concentration data. The candidate formula prepared by using the support vector machine is a high-powered expression (including kernel function) including each explanatory variable maximizing the boundary between groups. The candidate formula prepared by using the multiple regression analysis is a high-powered expression including each explanatory variable minimizing the sum of the distances from all concentration data. The candidate formula prepared by using the Cox regression analysis is a linear model including a logarithmic hazard ratio, and is a linear expression including each explanatory variable with a coefficient thereof maximizing the likelihood of the linear model. The candidate formula prepared by using the logistic regression analysis is a linear model expressing logarithmic odds of probability, and a linear expression including each explanatory variable maximizing the likelihood of the probability. The K-means method is a method of searching k pieces of neighboring concentration data in various groups, designating the group containing the greatest number of the neighboring points as its data-belonging group, and selecting the explanatory variable that makes the group to which input concentration data belong agree well with the designated group. The cluster analysis is a method of clustering (grouping) the points closest in entire concentration data. The decision tree is a method of ordering explanatory variables and predicting the group of concentration data from the pattern possibly held by the higher-ordered explanatory variable.

Returning to the description of the evaluation formula-preparing processing, the control device verifies (mutually verifies) the candidate formula prepared in step 1 based on a particular verifying method (step 2). The verification of the candidate formula is performed on each other to each candidate formula prepared in step 1.

In step 2, at least one of discrimination rate, sensitivity, specificity, information criterion, ROC_AUC (area under the curve in a receiver operating characteristic curve), and the like of the candidate formula may be verified by at least one of bootstrap method, holdout method, N-fold method, leave-one-out method, and the like. In this way, it is possible to prepare the candidate formula higher in predictability or reliability, by taking the index state information and the evaluation condition into consideration.

The discrimination rate is a rate in which a subject to be evaluated whose true state is negative (for example, the subject who has not experienced relapse in a certain period (e.g., one year) after blood collecting) is correctly evaluated as being negative by the evaluation method of the risk of relapse according to the present embodiment and a subject to be evaluated whose true state is positive (for example, the subject who has experienced relapse in a certain period (e.g., one year) after blood collecting) is correctly evaluated as being positive by the evaluation method of the risk of relapse according to the present embodiment. The sensitivity is a rate in which a subject to be evaluated whose true state is positive is correctly evaluated as being positive by the evaluation method of the risk of relapse according to the present embodiment. The specificity is a rate in which a subject to be evaluated whose true state is negative is correctly evaluated as being negative by the evaluation method of the risk of relapse according to the present embodiment. The Akaike information criterion is a criterion representing how observation data agrees with a statistical model, for example, in the regression analysis, and it is determined that the model in which the value defined by "-2×(maximum log-likelihood of statistical model)+2×(the number of free parameters of statistical model)" is smallest is the best. ROC_AUC (the area under the receiver operating characteristics curve) is defined as the area under the receiver operating characteristics curve (ROC) created by plotting (x, y)=(1-specificity, sensitivity) on two-dimensional coordinates. The value of ROC_AUC is 1 in perfect discrimination, and the closer this value is to 1, the higher the discriminative characteristic. The predictability is the average of discrimination rates, sensitivities, or specificities obtained by repeating the validation of the candidate formula. The robustness refers to the variance of discrimination rates, sensitivities, or specificities obtained by repeating the validation of the candidate formula.

Returning to the description of the evaluation formula-preparing processing, the control device selects a combination of the concentration data contained in the index state information used in preparing the candidate formula, by selecting an explanatory variable of the candidate formula based on a predetermined explanatory variable-selecting method (step 3). The selection of the explanatory variable may be performed on each candidate formula prepared in step 1. In this way, it is possible to select the explanatory variable of the candidate formula properly. The step 1 is executed once again by using the index state information including the concentration data selected in step 3.

In step 3, the explanatory variable of the candidate formula may be selected based on at least one of stepwise method, best path method, local search method, and genetic algorithm from the verification result obtained in step 2.

The best path method is a method of selecting an explanatory variable by optimizing an evaluation index of the candidate formula while eliminating the explanatory variables contained in the candidate formula one by one.

Returning to the description of the evaluation formula-preparing processing, the control device prepares the evaluation formula by repeatedly performing steps 1, 2 and 3, and based on the verification results thus accumulated, selecting the candidate formula used as the evaluation formula from the candidate formulae (step 4). In the selection of the candidate formula, there are cases where the optimum formula is selected from the candidate formulae prepared in the same formula-preparing method or the optimum formula is selected from all candidate formulae.

As described above, in the evaluation formula-preparing processing, the processing for the preparation of the candidate formulae, the verification of the candidate formulae, and the selection of the explanatory variables in the candidate formulae are performed based on the index state information in a series of operations in a systematized manner, whereby the evaluation formula most appropriate for evaluating the risk of relapse can be prepared. In other words, in the evaluation formula-preparing processing, the concentration of the substance in blood including at least one of His, Alb, Hb, and Glu is used in multivariate statistical analysis, and for selecting the optimum and robust combination of the explanatory variables, the explanatory variable-selecting method is combined with cross-validation to extract the evaluation formula having high evaluation performance.

### 2-2. System configuration

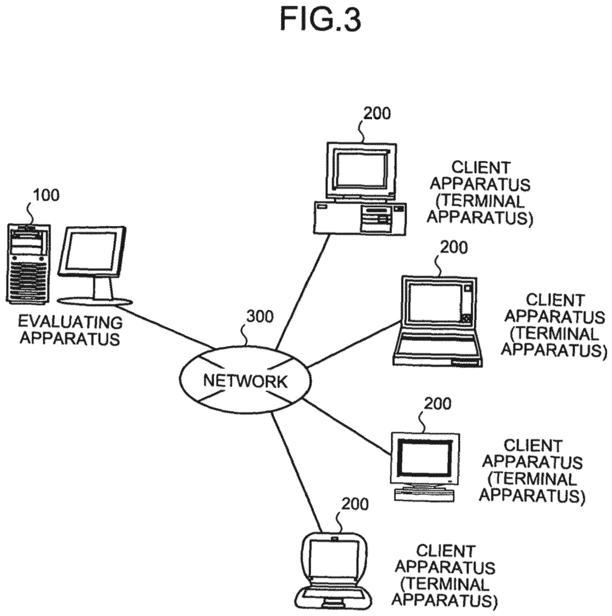

Hereinafter, the configuration of the evaluating system according to the second embodiment (hereinafter referred to sometimes as the present system) will be described with reference to FIGS. 3 to 14. This system is merely one example, and the present invention is not limited thereto. In particular, herein, when the risk of relapse is evaluated, a case of using the value of the evaluation formula or the converted value thereof is described as one example. However, for example, the concentration value of at least one of His, Alb, Hb, and Glu or the converted value thereof (for example, the concentration standard score) may be used.

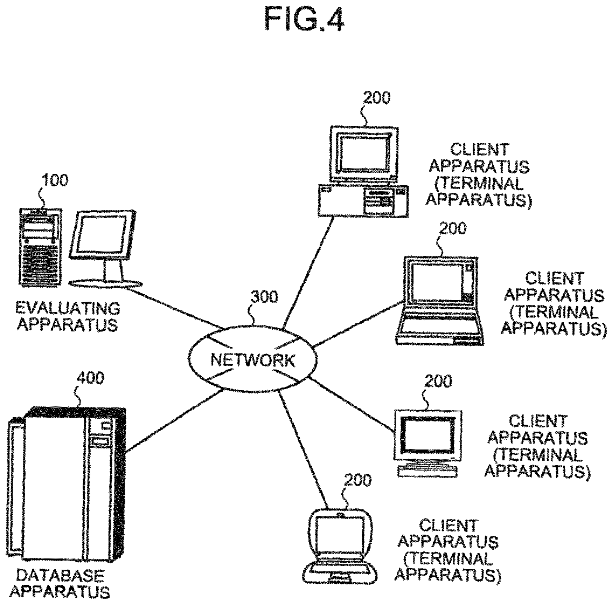

First, an entire configuration of the present system will be described with reference to FIGS. 3 and 4. FIG. 3 is a diagram showing an example of the entire configuration of the present system. FIG. 4 is a diagram showing another example of the entire configuration of the present system. As shown in FIG. 3, the present system is constituted in which the evaluating apparatus 100 that evaluates the risk of relapse for the individual in a remission phase of UC as the subject and the client apparatus 200 (corresponding to the terminal apparatus of the present invention) that provides the concentration data of the individual in a remission phase of UC on the concentration value of the substance in blood including at least one of His, Alb, and Hb in blood, are communicatively connected to each other via a network 300.

In the present system as shown in FIG. 4, in addition to the evaluating apparatus 100 and the client apparatus 200, the database apparatus 400 storing, for example, the index state information used in preparing the evaluation formula and the evaluation formula used in evaluating the risk of relapse in the evaluating apparatus 100, may be communicatively connected via the network 300. In this configuration, for example, information that may be helpful in knowing the risk of relapse is provided via the network 300 from the evaluating apparatus 100 to the client apparatuses 200 and the database apparatus 400, or from the client apparatuses 200 and the database apparatus 400 to the evaluating apparatus 100. The information that may be helpful in knowing the risk of relapse is, for example, information on the measured value of a particular item as to the state of the risk of relapse of organisms in a remission phase of UC including human. The information that may be helpful in knowing the risk of relapse is generated in the evaluating apparatus 100, the client apparatus 200, or other apparatuses (e.g., various measuring apparatuses) and stored mainly in the database apparatus 400.

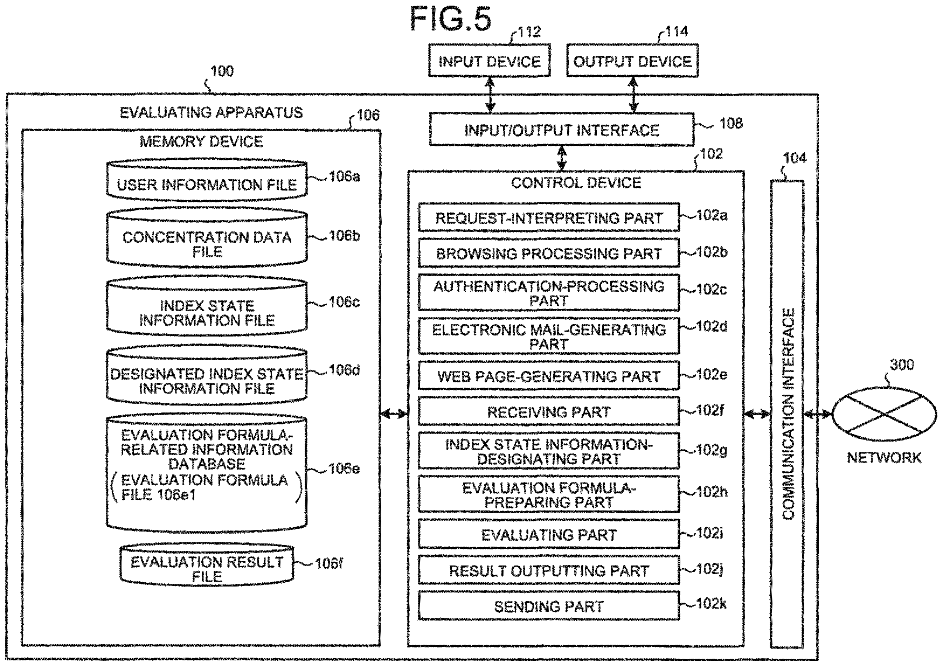

Now, the configuration of the evaluating apparatus 100 in the present system will be described with reference to FIGS. 5 to 12. FIG. 5 is a block diagram showing an example of the configuration of the evaluating apparatus 100 in the present system, showing conceptually only the region relevant to the present invention.

The evaluating apparatus 100 includes (I) a control device 102, such as CPU (Central Processing Unit), that integrally controls the evaluating apparatus, (II) a communication interface 104 that connects the evaluating apparatus to the network 300 communicatively via communication apparatuses such as a router and wired or wireless communication lines such as a private line, (III) a memory device 106 that stores various databases, tables, files and others, and (IV) an input/output interface 108 connected to an input device 112 and an output device 114, and these parts are connected to each other communicatively via any communication channel. The evaluating apparatus 100 may be present together with various analyzers (e.g., amino acid analyzer) in a same housing. For example, the evaluating apparatus 100 may be a compact analyzing device including components (hardware and software) that calculate (measure) the concentration value of the predetermined substance in blood including at least one of His, Alb, and Hb in blood and output (e.g., print or display on a monitor) the calculated concentration value, wherein the compact analyzing device is characterized by further including the evaluating part 102i described later, and using the components to output results obtained by the evaluating part 102i.

The memory device 106 is a storage means, and examples thereof include a memory apparatus such as RAM (Random Access Memory) and ROM (Read Only Memory), a fixed disk drive such as a hard disk, a flexible disk, and an optical disk. The memory device 106 stores computer programs giving instructions to the CPU for various processings, together with OS (Operating System). As shown in the figure, the memory device 106 stores the user information file 106a, the concentration data file 106b, the index state information file 106c, the designated index state information file 106d, an evaluation formula-related information database 106e, and the evaluation result file 106f.

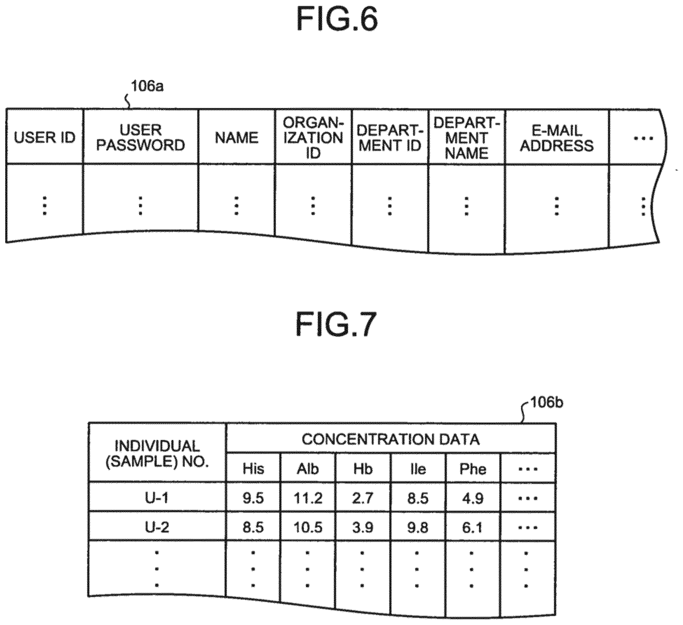

The user information file 106a stores user information on users. FIG. 6 is a chart showing an example of information stored in the user information file 106a. As shown in FIG. 6, the information stored in the user information file 106a includes a user ID (identification) for identifying a user uniquely, a user password for authentication of the user, a user name, an organization ID for uniquely identifying an organization of the user, a department ID for uniquely identifying a department of the user organization, a department name, and an electronic mail address of the user that are correlated to one another.

Returning to FIG. 5, the concentration data file 106b stores the concentration data on the concentration value of the substance in blood including at least one of His, Alb, and Hb in blood. FIG. 7 is a chart showing an example of information stored in the concentration data file 106b. As shown in FIG. 7, the information stored in the concentration data file 106b includes an individual number for uniquely identifying the individual (sample) as the subject and the concentration data that are correlated to one another. In FIG. 7, the concentration data is assumed to be numerical values, i.e., on a continuous scale, but the concentration data may be expressed on a nominal scale or an ordinal scale. In the case of the nominal or ordinal scale, any number may be allocated to each state for analysis. The concentration data may be combined with the value related to other biological information (see above).

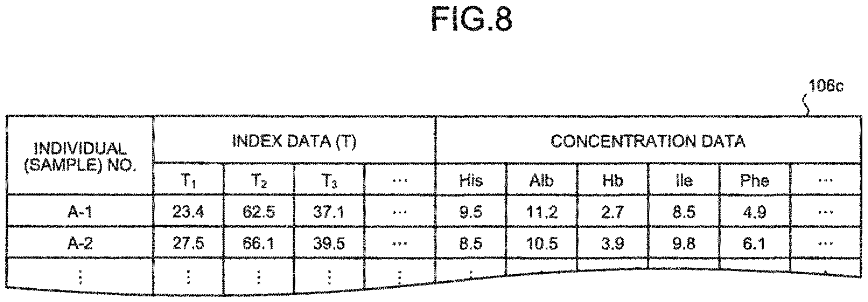

Returning to FIG. 5, the index state information file 106c stores the index state information used in preparing the evaluation formula. FIG. 8 is a chart showing an example of information stored in the index state information file 106c. As shown in FIG. 8, the information stored in the index state information file 106c includes the individual number, the index data (T) on the state of the predictive index of relapse of UC (index T₁, index T₂, index T₃ ...), and the concentration data that are correlated to one another. In FIG. 8, the index data and the concentration data are assumed to be numerical values, i.e., on a continuous scale, but the index data and the concentration data may be expressed on a nominal scale or an ordinal scale. In the case of the nominal or ordinal scale, any number may be allocated to each state for analysis. The index data is, for example, a known index of a prediction of relapse of UC, and numerical data may be used.

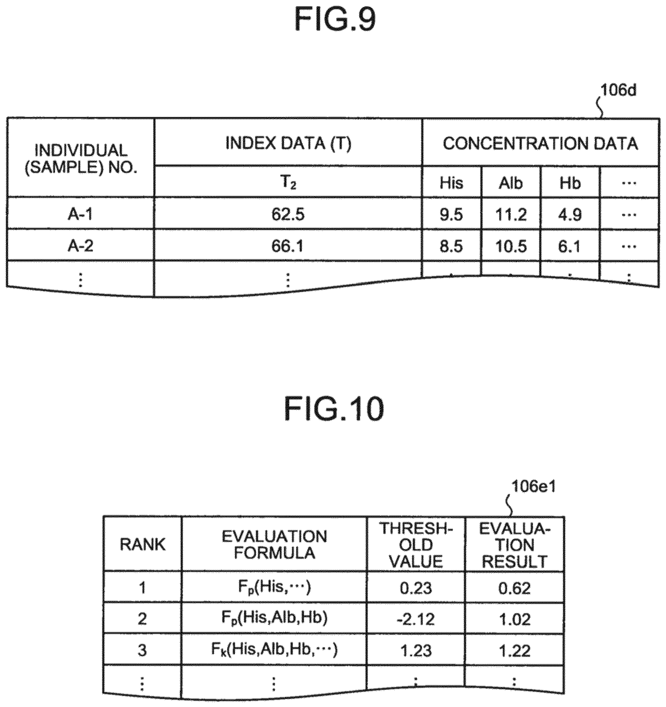

Returning to FIG. 5, the designated index state information file 106d stores the index state information designated in an index state information-designating part 102g described below. FIG. 9 is a chart showing an example of information stored in the designated index state information file 106d. As shown in FIG. 9, the information stored in the designated index state information file 106d includes the individual number, the designated index data, and the designated concentration data that are correlated to one another.

Returning to FIG. 5, the evaluation formula-related information database 106e is composed of the evaluation formula file 106e1 storing the evaluation formula prepared in an evaluation formula-preparing part 102h described below.

The evaluation formula file 106e1 stores the evaluation formulae. FIG. 10 is a chart showing an example of information stored in the evaluation formula file 106e1. As shown in FIG. 10, the information stored in the evaluation formula file 106e1 includes a rank, the evaluation formula (e.g., Fp (His,...), Fₚ (His, Alb, Hb), Fₖ (His, Alb, Hb,...) in FIG. 10), a threshold corresponding to each formula-preparing method, and the verification result of each evaluation formula (e.g., the evaluation value of each evaluation formula) that are correlated to one another.

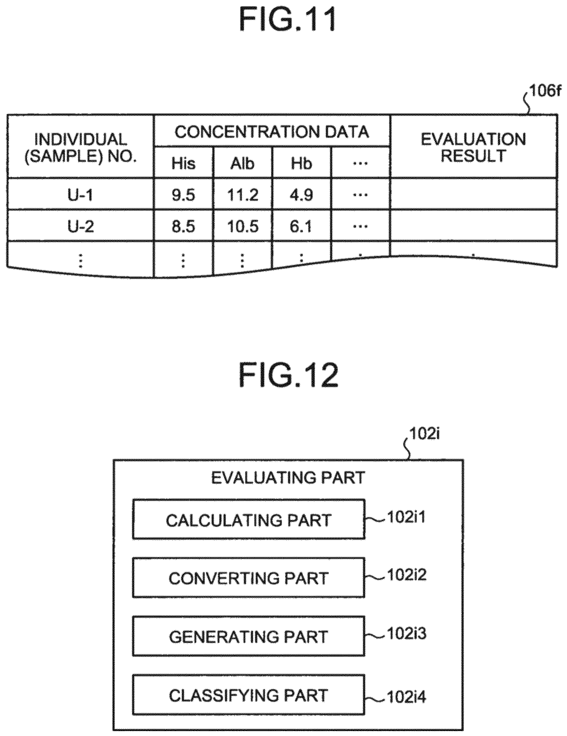

Returning to FIG. 5, the evaluation result file 106f stores the evaluation results obtained in the evaluating part 102i described below. FIG. 11 is a chart showing an example of information stored in the evaluation result file 106f. The information stored in the evaluation result file 106f includes the individual number for uniquely identifying the individual (sample) as the subject, the previously obtained concentration data of the individual, and the evaluation result on the state of the risk of relapse (for example, the value of the evaluation formula calculated by a calculating part 102i1 described below, the converted value of the evaluation formula by a converting part 102i2 described below, the positional information generated by a generating part 102i3 described below, or the classification result obtained by a classifying part 102i4 described below), that are correlated to one another.

Returning to FIG. 5, the memory device 106 stores various Web data for providing the client apparatuses 200 with web site information, CGI programs, and others as information other than the information described above. The Web data include data for displaying the Web pages described below and others, and the data are generated as, for example, a HTML (HyperText Markup Language) or XML (Extensible Markup Language) text file. Files for components and files for operation for generation of the Web data, and other temporary files, and the like are also stored in the memory device 106. In addition, the memory device 106 may store as needed sound files of sounds for transmission to the client apparatuses 200 in WAVE format or AIFF (Audio Interchange File Format) format and image files of still images or motion pictures in JPEG (Joint Photographic Experts Group) format or MPEG2 (Moving Picture Experts Group phase 2) format.

The communication interface 104 allows communication between the evaluating apparatus 100 and the network 300 (or a communication apparatus such as a router). Thus, the communication interface 104 has a function to communicate data via a communication line with other terminals.

The input/output interface 108 is connected to the input device 112 and the output device 114. A monitor (including a home television), a speaker, or a printer may be used as the output device 114 (hereinafter, the output device 114 may be described as the monitor 114). A keyboard, a mouse, a microphone, or a monitor functioning as a pointing device together with a mouse may be used as the input device 112.

The control device 102 has an internal memory storing, for example, control programs such as OS (Operating System), programs for various processing procedures, and other needed data, and performs various information processings according to these programs. As shown in the figure, the control device 102 includes mainly a request-interpreting part 102a, a browsing processing part 102b, an authentication-processing part 102c, an electronic mail-generating part 102d, a Web page-generating part 102e, a receiving part 102f, the index state information-designating part 102g, the evaluation formula-preparing part 102h, the evaluating part 102i, a result outputting part 102j and a sending part 102k. The control device 102 performs data processings such as removal of data including defective, removal of data including many outliers, and removal of explanatory variables for the defective-including data in the index state information transmitted from the database apparatus 400 and in the concentration data transmitted from the client apparatus 200.

The request-interpreting part 102a interprets the requests transmitted from the client apparatus 200 or the database apparatus 400 and sends the requests to other parts in the control device 102 according to results of interpreting the requests. Upon receiving browsing requests for various screens transmitted from the client apparatus 200, the browsing processing part 102b generates and transmits web data for these screens. Upon receiving authentication requests transmitted from the client apparatus 200 or the database apparatus 400, the authentication-processing part 102c performs authentication. The electronic mail-generating part 102d generates electronic mails including various kinds of information. The Web page-generating part 102e generates Web pages for users to browse with the client apparatus 200.

The receiving part 102f receives, via the network 300, information (specifically, the concentration data, the index state information, the evaluation formula, etc.) transmitted from the client apparatus 200 and the database apparatus 400. The index state information-designating part 102g designates objective index data and objective concentration data in preparing the evaluation formula.

The evaluation formula-preparing part 102h generates the evaluation formula based on the index state information received in the receiving part 102f or the index state information designated in the index state information-designating part 102g. If the evaluation formulae are stored previously in a predetermined region of the memory device 106, the evaluation formula-preparing part 102h may generate the evaluation formula by selecting the desired evaluation formula out of the memory device 106. Alternatively, the evaluation formula-preparing part 102h may generate the evaluation formula by selecting and downloading the desired evaluation formula from another computer apparatus (e.g., the database apparatus 400) in which the evaluation formulae are previously stored.

Returning to FIG. 5, the evaluating part 102i evaluates the risk of relapse for the individual by calculating the value of the evaluation formula using the previously obtained formula (for example, the evaluation formula prepared by the evaluation formula-preparing part 102h or the evaluation formula received by the receiving part 102f) and the concentration value of at least one of His, Alb, and Hb included in the concentration data received by the receiving part 102f. The evaluating part 102i may evaluate the risk of relapse for the individual using the concentration value of at least one of His, Alb, Hb, and Glu or the converted value of the concentration value (for example, the concentration standard score).

Hereinafter, a configuration of the evaluating part 102i will be described with reference to FIG. 12. FIG. 12 is a block diagram showing the configuration of the evaluating part 102i, and only a part in the configuration related to the present invention is shown conceptually. The evaluating part 102i includes the calculating part 102i1, the converting part 102i2, the generating part 102i3, and the classifying part 102i4, additionally.

The calculating part 102i1 calculates the value of the evaluation formula using the concentration value of at least one of His, Alb, and Hb and the evaluation formula including the explanatory variable to be substituted with the concentration value of at least one of His, Alb, and Hb. The evaluating part 102i may store the value of the evaluation formula calculated by the calculating part 102i1 as the evaluation result in a predetermined region of the evaluation result file 106f.

The converting part 102i2 converts the value of the evaluation formula calculated by the calculating part 102i1, for example, by the conversion method described above. The evaluating part 102i may store the converted value by the converting part 102i2 as the evaluation result in a predetermined region of the evaluation result file 106f. The converting part 102i2 may convert the concentration value of at least one of His, Alb, Hb, and Glu included in the concentration data, for example, by the conversion method described above.

The generating part 102i3 generates the positional information about the position of the predetermined mark on the predetermined scale visually presented on the display device such as a monitor or the physical medium such as paper, using the value of the formula calculated by the calculating part 102i1 or the converted value by the converting part 102i2 (the concentration value or the converted value of the concentration value may be used as swell). The evaluating part 102i may store the positional information generated by the generating part 102i3 as the evaluation result in a predetermined region of the evaluation result file 106f.

The classifying part 102i4 classifies the individual into any one of the categories defined at least considering the degree of the risk of relapse, using the value of the evaluation formula calculated by the calculating part 102i1 or the converted value by the converting part 102i2 (the concentration value or the converted value of the concentration value may be used as swell).

Returning to FIG. 5, the result outputting part 102j outputs, into the output device 114, for example, the processing results in each processing part in the control device 102 (including the evaluation results obtained by the evaluating part 102i).

The sending part 102k transmits the evaluation results to the client apparatus 200 that is a sender of the concentration data of the individual, and transmits the evaluation formulae prepared in the evaluating apparatus 100 and the evaluation results to the database apparatus 400.

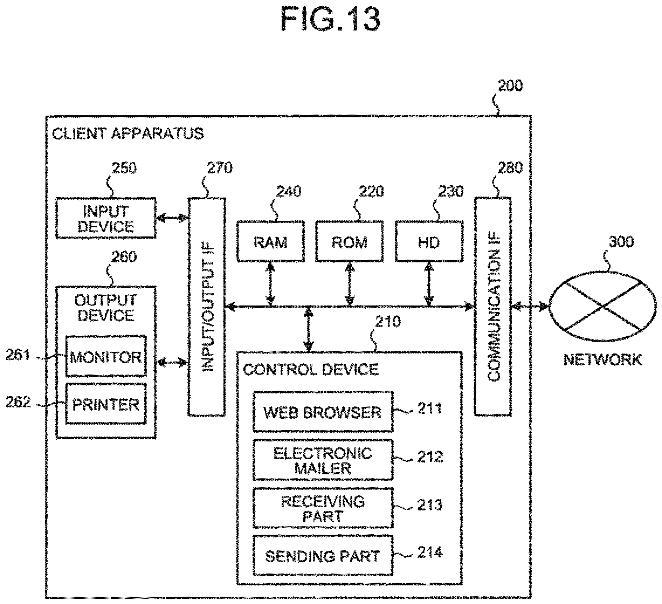

Hereinafter, a configuration of the client apparatus 200 in the present system will be described with reference to FIG. 13. FIG. 13 is a block diagram showing an example of the configuration of the client apparatus 200 in the present system, and only the part in the configuration relevant to the present invention is shown conceptually.

The client apparatus 200 includes a control device 210, ROM 220, HD (Hard Disk) 230, RAM 240, an input device 250, an output device 260, an input/output IF 270, and a communication IF 280 that are connected communicatively to one another through a communication channel.

The control device 210 has a Web browser 211, an electronic mailer 212, a receiving part 213, and a sending part 214. The Web browser 211 performs browsing processings of interpreting Web data and displaying the interpreted Web data on a monitor 261 described below. The Web browser 211 may have various plug-in softwares, such as stream player, having functions to receive, display and feedback streaming images. The electronic mailer 212 sends and receives electronic mails using a particular protocol (e.g., SMTP (Simple Mail Transfer Protocol) or POP3 (Post Office Protocol version 3)). The receiving part 213 receives various kinds of information, such as the evaluation results transmitted from the evaluating apparatus 100, via the communication IF 280. The sending part 214 sends various kinds of information such as the concentration data of the individual, via the communication IF 280, to the evaluating apparatus 100.

The input device 250 is, for example, a keyboard, a mouse, or a microphone. The monitor 261 described below also functions as a pointing device together with a mouse. The output device 260 is an output means for outputting information received via the communication IF 280, and includes the monitor 261 (including home television) and a printer 262. In addition, the output device 260 may have a speaker or the like additionally. The input/output IF 270 is connected to the input device 250 and the output device 260.

The communication IF 280 connects the client apparatus 200 to the network 300 (or communication apparatus such as a router) communicatively. In other words, the client apparatus 200 is connected to the network 300 via a communication apparatus such as a modem, TA (Terminal Adapter) or a router, and a telephone line, or via a private line. In this way, the client apparatus 200 can access to the evaluating apparatus 100 by using a particular protocol.

The client apparatus 200 may be realized by installing softwares (including programs, data and others) for a Web data-browsing function and an electronic mail-processing function to an information processing apparatus (for example, an information processing terminal such as a known personal computer, a workstation, a family computer, Internet TV (Television), PHS (Personal Handyphone System) terminal, a mobile phone terminal, a mobile unit communication terminal, or PDA (Personal Digital Assistants)) connected as needed with peripheral devices such as a printer, a monitor, and an image scanner.

All or a part of processings of the control device 210 in the client apparatus 200 may be performed by CPU and programs read and executed by the CPU. Computer programs for giving instructions to the CPU and executing various processings together with the OS (Operating System) are recorded in the ROM 220 or HD 230. The computer programs, which are executed as they are loaded in the RAM 240, constitute the control device 210 with the CPU. The computer programs may be stored in application program servers connected via any network to the client apparatus 200, and the client apparatus 200 may download all or a part of them as needed. All or any part of processings of the control device 210 may be realized by hardware such as wired-logic.

The control device 210 may include an evaluating part 210a (including a calculating part 210a1, a converting part 210a2, a generating part 210a3, and a classifying part 210a4) having the same functions as the functions of the evaluating part 102i in the control device 102 of the evaluating apparatus 100. When the control device 210 includes the evaluating part 210a, the evaluating part 210a may convert the value of the formula in the converting part 210a2, generate the positional information corresponding to the value of the formula or the converted value (the concentration value or the converted value of the concentration value may be used as well) in the generating part 210a3, and classify the individual into any one of the categories using the value of the formula or the converted value (the concentration value or the converted value of the concentration value may be used as well) in the classifying part 210a4, in accordance with information included in the evaluation result transmitted from the evaluating apparatus 100.

Hereinafter, the network 300 in the present system will be described with reference to FIGS. 3 and 4. The network 300 has a function to connect the evaluating apparatus 100, the client apparatuses 200, and the database apparatus 400 mutually, communicatively to one another, and is for example the Internet, an intranet, or LAN (Local Area Network (including both wired and wireless)). The network 300 may be VAN (Value Added Network), a personal computer communication network, a public telephone network (including both analog and digital), a leased line network (including both analog and digital), CATV (Community Antenna Television) network, a portable switched network or a portable packet-switched network (including IMT2000 (International Mobile Telecommunication 2000) system, GSM (registered trademark) (Global System for Mobile Communications) system, or PDC (Personal Digital Cellular)/PDC-P system), a wireless calling network, a local wireless network such as Bluetooth (registered trademark), PHS network, a satellite communication network (including CS (Communication Satellite), BS (Broadcasting Satellite), ISDB (Integrated Services Digital Broadcasting), and the like), or the like.

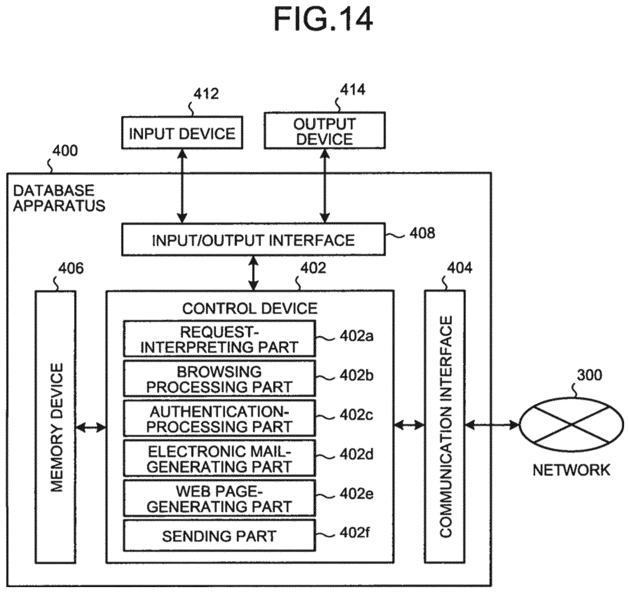

Hereinafter, the configuration of the database apparatus 400 in the present system will be described with reference to FIG. 14. FIG. 14 is a block diagram showing an example of the configuration of the database apparatus 400 in the present system, showing conceptually only the region relevant to the present invention.

The database apparatus 400 has functions to store, for example, the index state information used in preparing the evaluation formulae in the evaluating apparatus 100 or the database apparatus, the evaluation formulae prepared in the evaluating apparatus 100, and the evaluation results obtained in the evaluating apparatus 100. As shown in FIG. 14, the database apparatus 400 includes (I) a control device 402, such as CPU, that integrally controls the database apparatus, (II) a communication interface 404 connecting the database apparatus to the network 300 communicatively via communication apparatuses such as a router and wired or wireless communication circuits such as a private line, (III) a memory device 406 storing various databases, tables, files (for example, files for Web pages) and others, and (IV) an input/output interface 408 connected to an input device 412 and an output device 414, and these parts are connected communicatively to each other via any communication channel.

The memory device 406 is a storage means, and, examples thereof include a memory apparatus such as RAM or ROM, a fixed disk drive such as a hard disk, a flexible disk, and an optical disk. The memory device 406 stores, for example, various programs used in various processings. The communication interface 404 allows communication between the database apparatus 400 and the network 300 (or a communication apparatus such as a router). Thus, the communication interface 404 has a function to communicate data via a communication line with other terminals. The input/output interface 408 is connected to the input device 412 and the output device 414. A monitor (including a home television), a speaker, or a printer may be used as the output device 414 (hereinafter, the output device 414 may be described as the monitor 414). A keyboard, a mouse, a microphone, or a monitor functioning as a pointing device together with a mouse may be used as the input device 412.

The control device 402 has an internal memory storing, for example, control programs such as OS (Operating System), programs for various processing procedures, and other needed data, and performs various information processings according to these programs. As shown in the figure, the control device 402 includes mainly a request-interpreting part 402a, a browsing processing part 402b, an authentication-processing part 402c, an electronic mail-generating part 402d, a Web page-generating part 402e, and a sending part 402f.

The request-interpreting part 402a interprets the requests transmitted from the evaluating apparatus 100 and sends the requests to other parts in the control device 402 according to results of interpreting the requests. Upon receiving browsing requests for various screens transmitted from the evaluating apparatus 100, the browsing processing part 402b generates and transmits web data for these screens. Upon receiving authentication requests transmitted from the evaluating apparatus 100, the authentication-processing part 402c performs authentication. The electronic mail-generating part 402d generates electronic mails including various kinds of information. The Web page-generating part 402e generates Web pages for users to browse with the client apparatus 200. The sending part 402f transmits various kinds of information such as the index state information and the evaluation formulae to the evaluating apparatus 100.

In the present description, the evaluating apparatus 100 executes the reception of the concentration data, the calculation of the value of the evaluation formula, the classification of the individual into the category, and the transmission of the evaluation results, while the client apparatus 200 executes the reception of the evaluation results, described as an example. However, when the client apparatus 200 includes the evaluating unit 210a, the evaluating apparatus 100 only has to execute the calculation of the value of the evaluation formula. For example, the conversion of the value of the evaluation formula, the generation of the positional information, and the classification of the individual into the category may be appropriately shared between the evaluating apparatus 100 and the client apparatus 200.

For example, when the client apparatus 200 receives the value of the evaluation formula from the evaluating apparatus 100, the evaluating unit 210a may convert the value of the evaluation formula in the converting unit 210a2, generate the positional information corresponding to the value of the evaluation formula or the converted value in the generating unit 210a3, and classify the individual into any one of the categories for the risk of relapse using the value of the evaluation formula or the converted value in the classifying unit 210a4.

For example, when the client apparatus 200 receives the converted value from the evaluating apparatus 100, the evaluating unit 210a may generate the positional information corresponding to the converted value in the generating unit 210a3, and classify the individual into any one of the categories for the risk of relapse using the converted value in the classifying unit 210a4.

When the client apparatus 200 receives the value of the evaluation formula or the converted value and the positional information from the evaluating apparatus 100, the evaluating unit 210a may classify the individual into any one of the categories for the risk of relapse using the value of the evaluation formula or the converted value in the classifying unit 210a4.

### 2-3. Other embodiments

In addition to the second embodiment described above, the evaluating apparatus, the evaluating method, the evaluating program product, the evaluating system, and the terminal apparatus according to the present invention can be practiced in various different embodiments within the technological scope of the claims.

Of the processings described in the second embodiment, all or a part of the processings described as automatically performed ones may be manually performed, or all or a part of the processings described as manually performed ones may be also automatically performed by known methods.

In addition, the processing procedures, the control procedures, the specific names, the information including parameters such as registered data of various processings and retrieval conditions, the screen examples, and the database configuration shown in the description and the drawings may be arbitrarily modified unless otherwise specified.

The components of the evaluating apparatus 100 shown in the figures are functionally conceptual and therefore not be physically configured as shown in the figures.

For example, for the operational functions provided in the evaluating apparatus 100, in particular, for the operational functions performed in the control device 102, all or part thereof may be implemented by the CPU (Central Processing Unit) and programs interpreted and executed in the CPU, or may be implemented by wired-logic hardware. The program is recorded in a non-transitory tangible computer-readable recording medium including programmed instructions for making an information processing apparatus execute the evaluating method according to the present invention, and is mechanically read as needed by the evaluating apparatus 100. More specifically, computer programs to give instructions to the CPU in cooperation with the OS (operating system) to perform various processes are recorded in the memory device 106 such as ROM or a HDD (hard disk drive). The computer programs are executed by being loaded to RAM, and form the control unit in cooperation with the CPU.

The computer programs may be stored in an application program server connected to the evaluating apparatus 100 via an arbitrary network, and all or part thereof can be downloaded as necessary.

The evaluating program according to the present invention may be stored in the non-transitory tangible computer-readable recording medium, or can be configured as a program product. The "recording medium" mentioned here includes any "portable physical medium" such as a memory card, a USB (universal serial bus) memory, an SD (secure digital) card, a flexible disk, a magneto-optical disc, ROM, EPROM (erasable programmable read only memory), EEPROM (registered trademark) (electronically erasable and programmable read only memory), CD-ROM (compact disk read only memory), MO (magneto-optical disk), DVD (digital versatile disk), and Blu-ray (registered trademark) Disc.

The "program" mentioned here is a data processing method described in an arbitrary language or description method, and therefore any form such as a source code and a binary code is acceptable. The "program" is not necessarily limited to a program configured as a single unit, and, therefore, includes those dispersively configured as a plurality of modules and libraries and those in which the function of the program is achieved in cooperation with separate programs represented as OS (operating system). Any known configuration and procedures can be used as a specific configuration and reading procedure to read a recording medium by each apparatus shown in the embodiments, an installation procedure after the reading, and the like.

The various databases and the like stored in the memory device 106 is a storage unit such as a memory device such as RAM and ROM, a fixed disk drive such as a hard disk, a flexible disk, or an optical disc. The memory device 106 stores therein various programs, tables, databases, files for Web (World Wide Web) pages, and the like used to perform various processes and to provide Web sites.

The evaluating apparatus 100 may be configured as an information processing apparatus such as known personal computer and work station, or may be configured as the information processing apparatus connected to an arbitrary peripheral device. The evaluating apparatus 100 may be provided by installing software (including the programs and the data, etc.) to cause the information processing apparatus to implement the evaluating method according to the present invention.

Furthermore, a specific configuration of dispersion or integration of the apparatuses is not limited to the shown one. The apparatuses can be configured by functionally or physically dispersing or integrating all or part of the apparatuses in arbitrary units according to various types of additions or the like or according to functional loads. In other words, the embodiments may be implemented in arbitrary combinations thereof or an embodiment may be selectively implemented.

### Example 1

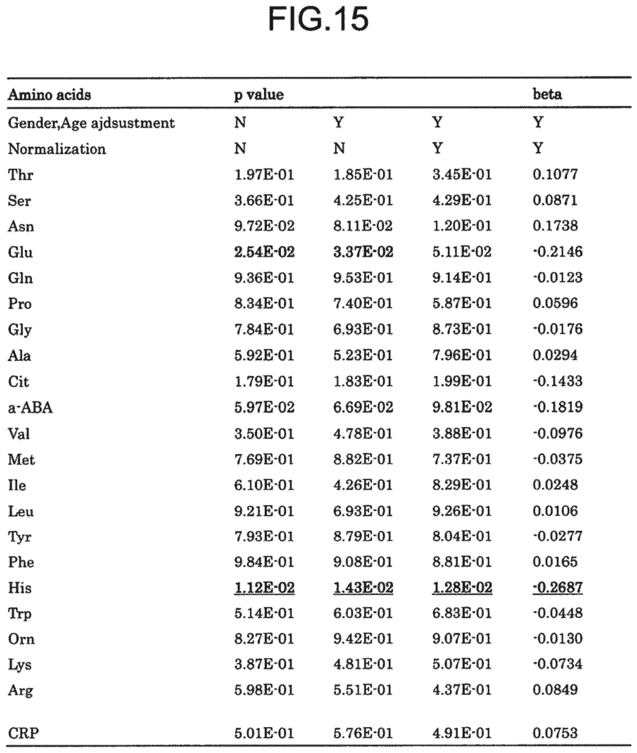

The blood samples were obtained under consent from the patients in a remission phase of UC (the patients with UC are confirmed that the Lichtiger CAI of them are less than 5), and relapse in the patients were observed for a year after the blood collecting (355 patients in total). The criteria of relapse were whether the total score of CAI reaches or exceeds five in one year after the blood collecting. The concentration values of amino acids (µM) were measured by the measuring method of (A). The Cox regression equation is estimated for each concentration value under the assumption that the feature of proportional hazard is established on the concentration value. Note that, in a case of using a regression equation, estimation was conducted for each of the following ways, that is, two ways including a case where each concentration value is an explanatory variable and a case where each concentration value standardized by Box-Cox conversion is an explanatory variable, and other two ways including a case where age and gender are added to explanatory values as potential confounding factors to be adjusted and a case where age and gender are not added to explanatory values, in a short, 2×2=4 ways in total. FIG. 15 shows the results of evaluating the association of the value obtained by substituting the respective concentration value and, as necessary, the values of age and gender into the estimated Cox regression equation, to relapse. Note that, in FIG. 15, the beta in the table means a partial regression coefficient.

According to the results, histidine and glutamic acid were significantly associated with a relapse event (p value was less than 0.05). By contrast, an inflammation index, or CRP (mg/dl) was not associated with relapse (p value was 0.05 or more). Among the amino acids, significant association of histidine with relapse was observed even when the adjustment was conducted by age and gender and the distribution of the concentration was adjusted by the Cox-Box conversion.

### Example 2

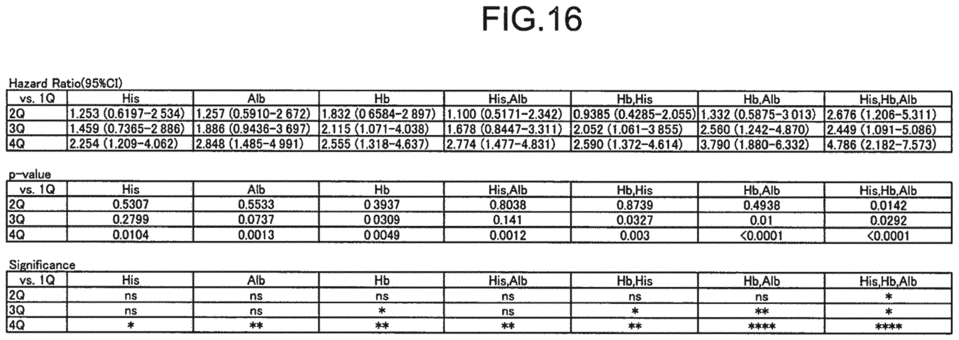

The blood samples of Example 1 were used. The concentration value of the histidine (µM) was measured by the measuring method of (A). A nutrition index, or the concentration value of the albumin (g/dL) was measured by the BCG method. An anemia index, or the concentration value of the hemoglobin (g/dL) was measured by the SLS-Hb method. With respect to the following seven patterns, the Cox regression equation is estimated for each concentration value under the assumption that the feature of proportional hazard is established on the concentration value. Note that, in a case of using a regression equation, the concentration values of the histidine, the albumin, and the hemoglobin, independently or as an optional-combination, were used as explanatory variables. In addition, the age and gender of each patient were also used as explanatory variables, as potential confounding factors to be adjusted. Next, the group of patients was divided into quartiles for each pattern by using the value obtained by substituting the respective concentration values and the values of age and gender into the estimated Cox regression equation. FIG. 16 shows the results of comparing the survival curves of the respective groups.

According to the result, as for the histidine (His), the hazard ratio of the fourth quartile to the first quartile for relapse was 2.254. As for the albumin (Alb), the hazard ratio of the fourth quartile to the first quartile for relapse was 2.848. As for the hemoglobin (Hb), the hazard ratio of the fourth quartile to the first quartile for relapse was 2.555. As for the combination of His and Alb, the hazard ratio of the fourth quartile to the first quartile for relapse was 2.774. As for the combination of His and Hb, the hazard ratio of the fourth quartile to the first quartile for relapse was 2.590. As for the combination of Alb and Hb, the hazard ratio of the fourth quartile to the first quartile for relapse was 3.790. As for the combination of His, Hb, and Alb, the hazard ratio of the fourth quartile to the first quartile for relapse was 4.786. As discussed above, the results indicated that the relapse of a patient in a remission phase of UC can be predicted by using histidine, albumin, and hemoglobin, independently or as an optional-combination, as an explanatory variable of the evaluation formula.

### INDUSTRIAL APPLICABILITY

As described above, the present invention may be practiced widely in many industrial fields, in particular in pharmaceutical, food, and medical fields, and is extremely useful especially in predicting relapse of a patient with UC in a remission phase.

### EXPLANATION OF LETTERS OR NUMERALS

100 Evaluating apparatus
102 Control device
102a Request-interpreting part
102b Browsing processing part
102c Authentication-processing part
102d Electronic mail-generating part
102e Web page-generating part
102f Receiving part
102g Index state information-designating part
102h Evaluation formula-preparing part
102i Evaluating part
102i1 Calculating part
102i2 Converting part
102i3 Generating part
102i4 Classifying part
102j Result outputting part
102k Sending part
104 Communication interface
106 Memory device
106a User information file
106b Concentration data file
106c Index state information file
106d Designated index state information file
106e Evaluation formula-related information database
106e1 Evaluation formula file
106f Evaluation result file
108 Input/output interface
112 Input device
114 Output device
200 Client apparatus (terminal apparatus (information communication terminal apparatus))
300 Network
400 Database apparatus

## Claims

1. An evaluating method comprising:
an evaluating step of evaluating a future risk of relapse of ulcerative colitis for a subject to be evaluated having ulcerative colitis in a remission phase using a concentration value of histidine, albumin, hemoglobin, or glutamic acid in blood of the subject.

2. The evaluating method according to claim 1, wherein the evaluating step uses the concentration values of histidine and albumin, histidine and hemoglobin, or albumin and hemoglobin.

3. The evaluating method according to claim 2, wherein the evaluating step uses the concentration values of histidine, albumin, and hemoglobin.

4. The evaluating method according to claim 1, wherein the evaluating step evaluates the future risk of relapse of ulcerative colitis for the subject by calculating a value of a formula further using the formula including an explanatory variable to be substituted with the concentration value of histidine, albumin, or hemoglobin.

5. The evaluating method according to claim 4, wherein the formula includes the explanatory variables to be substituted with the concentration values of histidine and albumin, histidine and hemoglobin, or albumin and hemoglobin.

6. The evaluating method according to claim 5, wherein the formula includes the explanatory variables to be substituted with the concentration values of histidine, albumin, and hemoglobin.

7. An evaluating apparatus comprising a control unit, wherein the control unit includes:
an evaluating unit that evaluates a future risk of relapse of ulcerative colitis for a subject to be evaluated having ulcerative colitis in a remission phase using a concentration value of histidine, albumin, hemoglobin, or glutamic acid in blood of the subject.

8. An evaluating method executed by an information processing apparatus including a control unit, wherein the evaluating method comprises an evaluating step of evaluating a future risk of relapse of ulcerative colitis for a subject to be evaluated having ulcerative colitis in a remission phase using a concentration value of histidine, albumin, hemoglobin, or glutamic acid in blood of the subject, wherein the evaluating step is executed by the control unit.

9. An evaluating program product having a non-transitory tangible computer readable medium including programmed instructions for making an information processing apparatus including a control unit execute an evaluating method, wherein the evaluating method comprises an evaluating step of evaluating a future risk of relapse of ulcerative colitis for a subject to be evaluated having ulcerative colitis in a remission phase using a concentration value of histidine, albumin, hemoglobin, or glutamic acid in blood of the subject.

10. An evaluating system comprising an evaluating apparatus including a control unit and a terminal apparatus including a control unit to provide concentration data on a concentration value of histidine, albumin, hemoglobin, or glutamic acid in blood of a subject to be evaluated having ulcerative colitis in a remission phase that are connected to each other communicatively via a network, wherein
the control unit of the terminal apparatus includes:
a concentration data-sending unit that transmits the concentration data of the subject to the evaluating apparatus; and
a result-receiving unit that receives an evaluation result on a future risk of relapse of ulcerative colitis for the subject, transmitted from the evaluating apparatus, and
the control unit of the evaluating apparatus includes:
a concentration data-receiving unit that receives the concentration data of the subject transmitted from the terminal apparatus;
an evaluating unit that evaluates the future risk of relapse of ulcerative colitis for the subject using the concentration value of histidine, albumin, hemoglobin, or glutamic acid included in the concentration data of the subject received by the concentration data-receiving unit; and
a result-sending unit that transmits the evaluation result of the subject obtained by the evaluating unit to the terminal apparatus.

11. A terminal apparatus comprising a control unit,
wherein the control unit includes a result-obtaining unit that obtains an evaluation result on a future risk of relapse of ulcerative colitis for a subject to be evaluated having ulcerative colitis in a remission phase,
wherein the evaluation result is the result of evaluating the future risk of relapse of ulcerative colitis for the subject using a concentration value of histidine, albumin, hemoglobin, or glutamic acid in blood of the subject.

12. An evaluating apparatus comprising a control unit, being connected communicatively via a network to a terminal apparatus that provides concentration data on a concentration value of histidine, albumin, hemoglobin, or glutamic acid in blood of a subject to be evaluated having ulcerative colitis in a remission phase, wherein the control unit includes:
a concentration data-receiving unit that receives the concentration data of the subject transmitted from the terminal apparatus;
an evaluating unit that evaluates a future risk of relapse of ulcerative colitis for the subject using the concentration value of histidine, albumin, hemoglobin, or glutamic acid included in the concentration data of the subject received by the concentration data-receiving unit; and
a result-sending unit that transmits an evaluation result obtained by the evaluating unit to the terminal apparatus.
